# EUROPEAN PATENT APPLICATION

(11) **EP 4 129 338 A1**
(43) Date of publication of application: **08.02.2023**
(21) Application number: 21763528.3
(22) Date of filing: 02.03.2021
(51) Int. Cl.: A61K 45/00, A61P 9/10, C12N 15/113, A61K 31/7105, A61K 31/711, A61K 31/7115, A61K 31/712, A61K 31/7125, A61K 31/713

(54) **PREVENTION OR TREATMENT OF ANEURYSMS USING MIR-33B INHIBITOR**

(30) Priority: 02.03.2020 JP 2020034619
(71) Applicant: Mitsubishi Tanabe Pharma Corporation, Osaka-shi, Osaka 541-8505 (JP); Kyoto University, Kyoto-shi, Kyoto 606-8501 (JP); OSAKA UNIVERSITY, Suita-shi Osaka 565-0871 (JP); National Institutes of Biomedical Innovation, Health and Nutrition, Ibaraki-shi, Osaka 567-0085 (JP)
(72) Inventor: KOTERA, Jun, Osaka-shi, Osaka 541-8505 (JP); ONO, Koh, Kyoto-shi, Kyoto 606-8501 (JP); HORIE, Takahiro, Kyoto-shi, Kyoto 606-8501 (JP); YAMASAKI, Tomohiro, Kyoto-shi, Kyoto 606-8501 (JP); KOYAMA, Satoshi, Kyoto-shi, Kyoto 606-8501 (JP); OBIKA, Satoshi, Suita-shi, Osaka 565-0871 (JP); KASAHARA, Yuya, Ibaraki-shi, Osaka 567-0085 (JP)
(74) Representative: J A Kemp LLP
(86) International application number: PCT/JP2021/007822
(87) International publication number: WO 2021/177267

(57) **Abstract**

A prophylactic or therapeutic agent for an aneurysm comprising a miR-33b inhibiting substance, preferably an antisense oligonucleotide against miR-33b, as an active ingredient.

## Description

### [Technical Field]

The present invention relates to a prophylactic or therapeutic agent for aneurysms, which comprises a miR-33b inhibiting substance as an active ingredient.

### [Technical Background]

Prevalence of aortic aneurysms is increasing due to longevity and lifestyle changes. For example, abdominal aortic aneurysms is the third most common cause of cardiovascular mortality, accounting for 1% to 3% of all mortality in the male population aged 65 and over. Although early diagnosis by computed tomography scan is clinically possible, there has been no drug so far for preventing promotion of aortic aneurysms. Since aortic aneurysms asymptomatically progress and can result in fatal rupture, there is an urgent unmet need to develop a drug therapy for suppressing progression of aortic aneurysms. At present, increased extracellular matrix degradation due to protease activation, cell death of macrovascular smooth muscle cells, endothelial dysfunction, and the like are important steps for arterial wall inflammation, and it is thought that inflammation accompanying these causes media destruction and aortic wall fragility and is involved in formation and progression of aortic aneurysms (Non-Patent Document 1).

Drug development so far has focused on inhibiting extracellular matrix degradation, which is thought to cause aortic wall fragility. However, doxycycline, an inhibitor of matrix metalloproteinase, which is one of major groups of proteases in etiology of aortic aneurysms, showed no clear clinical effect on progression of aneurysms (Non-Patent Document 1).

A miRNA (microRNA) is a tiny single-stranded RNA that is encoded on a genome and eventually reaches a length of 19 - 25 bases through a multi-step production process, and negatively controls a target gene expression by binding to 3'-untranslated regions of multiple targeted messenger RNAs (mRNAs) to suppress translation or promote degradation thereof. Currently, 2000 or more types of miRNAs have been identified in humans, and it is said that 60% or more of human gene products are controlled by miRNAs. Further, miRNAs are involved in onset and progression of various biological phenomena and pathological conditions, and thus, are attracting attention as biomarkers and new therapeutic targets.

miR-33 (microRNA-33) has been found as a miRNA that targets an ATP-binding cassette transporter A1 (ABCA1) to regulate cholesterol metabolism and lower a high-density lipoprotein cholesterol (HDL-C) level. It has been reported that miR-33a and miR-33b are present in humans, whereas only miR-33 (corresponding to human miR-33a) is present in mice (Non-Patent Document 2).

It has been reported that, regarding a relationship between aneurysms and miR-33, knockout mice of miR-33 (corresponding to human miR-33a) were evaluated using an aneurysm model, and aneurysm formation was suppressed, and that macrophage accumulation and monocyte chemotactic protein-1 expression were suppressed (Non-Patent Document 3).

Further, it has been reported that in humans, comprehensive gene expression analysis data in blood samples of patients with cerebral aneurysms was used, and an expression level of IRS2, which is a target gene of miR-33b, was increased (Non-Patent Document 4). Since the expression of IRS2 is subjected to direct control (expression suppression) by miR-33b (Non-Patent Document 5), this phenomenon suggests that supplementation, rather than inhibition, of miR-33b is effective in suppressing human aneurysm formation.

Further, it has been reported that expression of miR-33-5p in abdominal aorta of patients with human abdominal aortic aneurysms is increased and that gene expression of MMP-2, MMP-9 and TNF-α is suppressed by an inhibitor of miR-33-5p in human monocyte-derived macrophage cell lines and cholesterol in foamed macrophages is reduced by promoting cholesterol excretion (Non-Patent Document 6). Here, a sequence of miR-33b is described as a sequence of miR-33-5p. However, in an expression analysis, no distinction is made between miR-33a and miR-33b, and further, it is not stated whether a target molecule of an inhibitor of miR-33-5p is human miR-33a or miR-33b. And, in this document, a therapeutic effect of abdominal aortic aneurysms is not shown.

Further, it has been reported that mice expressing human miR-33a or human miR-33b were prepared, and arteriosclerosis was worsened in mice expressing miR-33b. This suggests that miR-33b is involved in exacerbation of arteriosclerosis by increasing expression of miR-33b and decreasing production of HDL cholesterol in liver cells due to a high cholesterol dietary load (Non-Patent Document 2). It has been reported that macrophages are associated with aneurysms (Non-Patent Document 1). However, it has been reported that there is no difference in changes of expression levels of miR-33a and miR-33b in macrophages and there is also no functional difference (Non-Patent Document 2).

### [Related Art]

### [Non-Patent Documents]

[Non-Patent Document 1] The Journal of the Japanese Society of Internal Medicine 99, p237-244 (2010)
[Non-Patent Document 2] J Am Heart Assoc., 8 (13), e012609 (2019)
[Non-Patent Document 3] Arterioscler Thromb Vasc Biol., 37 (11), p2161-2170 (2017)
[Non-Patent Document 4] Med Sci Monit., 23, p4518-4525 (2017)
[Non-Patent Document 5] Proc Natl Acad Sci USA, 108 (22), 9232-7 (2011)
[Non-Patent Document 6] Perfusion., doi: 10.1177/0267659119850685 (2019)

### [Summary of the Invention]

### [Problems to Be Solved by the Invention]

The present invention aims at prevention and/or treatment of aneurysms using a drug effective for the prevention and treatment of aneurysms. More specifically, the present invention relates to overall treatment of aneurysms that have developed, and is intended to provide an aneurysm prophylactic and/or therapeutic agent capable of preventing development or expansion of aneurysms or capable of preventing rupture due to regression of aneurysms.

### [Means for Solving the Problems]

As a result of studies using inhibitors of miR-33a and miR-33b to analyze contribution of miR-33a and miR-33b to aortic aneurysms, the present inventors have found that a miR-33b inhibiting substance is useful for treatment, prevention, or alleviation of aneurysms, and thus have accomplished the present invention.

The present disclosure relates to, but is not limited to, the non-limiting numbered embodiments described in the following aspects [1] to [25].
[1] A prophylactic or therapeutic agent for an aneurysm comprising a miR-33b inhibiting substance as an active ingredient.
[2] The prophylactic or therapeutic agent according to [1], wherein said miR-33b inhibiting substance is a substance reducing miR-33b level.
[3] The prophylactic or therapeutic agent according to [2], wherein the substance reducing miR-33b level is a nucleic acid.
[4] The prophylactic or therapeutic agent according to [3], wherein the substance reducing miR-33b level is an antisense oligonucleotide, a siRNA or a shRNA against miR-33b.
[5] The prophylactic or therapeutic agent according to any one of [2] to [4], wherein a reducing rate of miR-33b level of the substance reducing miR-33b level is higher than a reducing rate of miR-33a level of the substance reducing miR-33b level.
[6] The prophylactic or therapeutic agent according to any one of [2] to [5], wherein the substance reducing miR-33b level is an antisense oligonucleotide against miR-33b.
[7] The prophylactic or therapeutic agent according to [6], wherein the antisense oligonucleotide against miR-33b has a base sequence that (1) is formed of 7 to 20 nucleotide residues and (2) is 90% or more complementary to an equal length portion of miR-33b having a base sequence of SEQ ID NO: 1, and does not have a mismatch with 9th and 10th nucleobases counting from a 5' end of a base sequence of miR-33b described in SEQ ID NO: 1:
   (SEQ ID NO: 1) GUGCAUUGCUGUUGCAUUGC
[8] The prophylactic or therapeutic agent according to [7], wherein the antisense oligonucleotide against miR-33b comprises a nucleobase sequence described in
   AACAGCA ATGCA (SEQ ID NO: 5)
   (wherein C may be a 5-methylcytosine (M)).
[9] The prophylactic or therapeutic agent according to [8], wherein the antisense oligonucleotide is a modified oligonucleotide.
[10] The prophylactic or therapeutic agent according to [9], wherein at least one nucleoside forming the modified oligonucleotide comprises a modified sugar.
[11] The prophylactic or therapeutic agent according to [10], wherein the modified sugar is selected from a sugar moiety of AmNA: 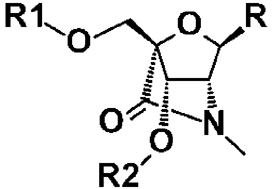 AmNA
   [wherein, R is a nucleobase, and R1 and R2 each independently indicate a phosphate group that may be substituted].
[12] The prophylactic or therapeutic agent according to [11], wherein the modified oligonucleotide is a modified oligonucleotide represented by the following formula:

   Aas Ads Mas Ads Gas Cds Aas Ads Tas Gds Mas Ad, wherein nucleobases are represented according to the following symbols:

   A = adenine, T = thymine, G = guanine, C = cytosine, M = 5-methylcytosine,
   sugar moieties are represented according to the following symbols:
      a = a sugar moiety of the above AmNA, d = 2'-deoxyribose,
   and internucleoside linkages are represented according to the following symbol:
      s = phosphorothioate.
[13] The prophylactic or therapeutic agent according to [12], wherein the modified oligonucleotide is a modified oligonucleotide represented by the following formula: 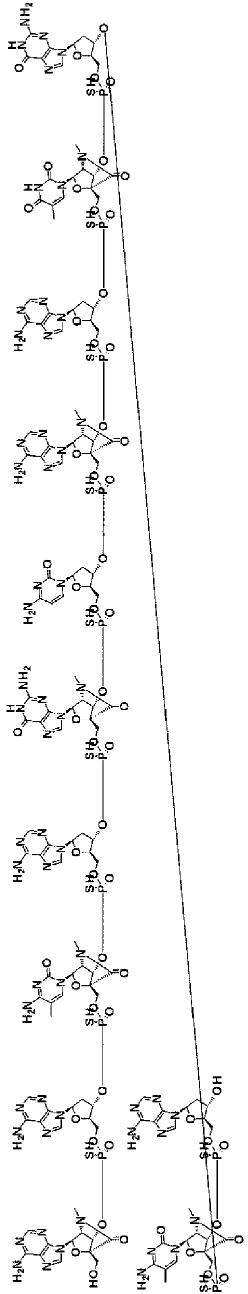
[14] The prophylactic or therapeutic agent according to any one of [1] to [13], wherein the aneurysm is an aortic aneurysm or a cerebral aneurysm.
[15] An antisense oligonucleotide against miR-33b comprising a nucleobase sequence described in AACAGCAATGCA (SEQ ID NO: 5).
[16] The antisense oligonucleotide according to [15], wherein the antisense oligonucleotide is a modified oligonucleotide.
[17] The antisense oligonucleotide according to [16], wherein at least one nucleoside forming the modified oligonucleotide comprises a modified sugar.
[18] The antisense oligonucleotide according to [17], wherein the modified sugar is selected from a sugar moiety of AmNA: 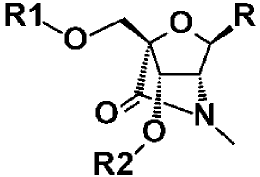 AmNA
   [wherein, R is a nucleobase, and R1 and R2 each independently indicate a phosphate group that may be substituted].
[19] The antisense oligonucleotide according to any one of [16] to [18], wherein at least one nucleoside forming the modified oligonucleotide comprises a modified nucleobase.
[20] The antisense oligonucleotide according to [19], wherein the modified nucleobase is a 5-methylcytosine.
[21] The antisense oligonucleotide according to any one of [16] to [20], wherein a modified internucleoside linkage of the modified oligonucleotide is a phosphorothioate internucleoside linkage.
[22] The antisense oligonucleotide according to any one of [16] to [21], wherein the modified oligonucleotide is a modified oligonucleotide represented by the following formula:

   Aas Ads Mas Ads Gas Cds Aas Ads Tas Gds Mas Ad,

   wherein nucleobases are represented according to the following symbols:
   A = adenine, T = thymine, G = guanine, C = cytosine, M = 5-methylcytosine,
   sugar moieties are represented according to the following symbols:
      a = a sugar moiety of the above AmNA, d = 2'-deoxyribose,
      and internucleoside linkages are represented according to the following symbol:
         s = phosphorothioate.
[23] The antisense oligonucleotide according to [22], wherein the modified oligonucleotide is represented by the following formula: 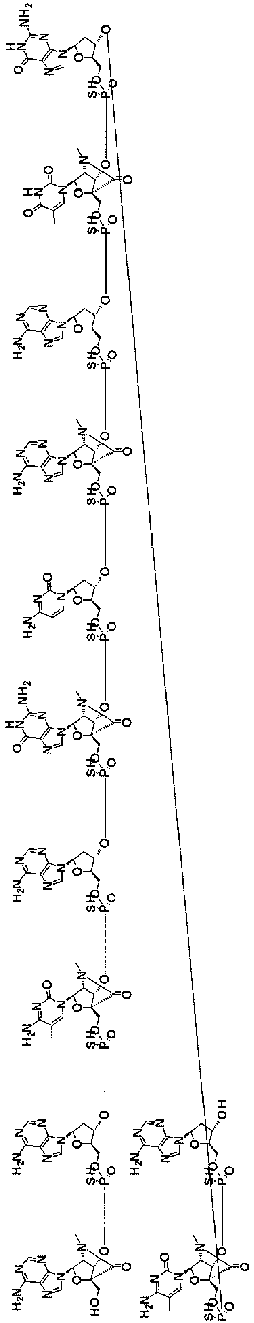
[24] A pharmaceutical composition comprising: the antisense oligonucleotide according to any one of claims 15 to 23 or a pharmaceutically acceptable salt thereof; and a pharmaceutically acceptable carrier.
[25] Use of a miR-33b inhibiting substance in manufacturing a prophylactic or therapeutic agent for an aneurysm.

### [Effects of the Invention]

A miR-33b inhibiting substance suppresses development or expansion of aneurysms and thus can be used as a prophylactic and/or therapeutic agent for aneurysms.

### [Brief Description of the Drawings]

[Fig. 1] Fig. 1 shows graphs showing results of in vivo administration tests evaluating effects of antisense oligonucleotides on expression levels of miR-33a (A) and miR-33b (B) in mouse aorta.
[Fig. 2] Fig. 2 shows graphs showing results of in vivo administration tests evaluating effects of antisense oligonucleotides on a MCP-1 protein expression level(A), a phosphorylated JNK1 protein expression level (B), and a JNK1 protein expression level (C) in mouse aorta.
[Fig. 3] Fig. 3 is a graph showing results of in vivo administration test evaluating effects of antisense oligonucleotides on a MMP-9 activity level in mouse aorta.
[Fig. 4] Fig. 4 shows graphs showing results of in vivo administration tests evaluating effects of antisense oligonucleotides on diameter enlargement (A) and extension (B) of aneurysms in a 1-week mouse aortic aneurysm model.
[Fig. 5] Fig. 5 shows graphs showing results of in vivo administration tests evaluating effects of antisense oligonucleotides on diameter enlargement (A, C) and extension (B, C) of aneurysms in a 6-week mouse aortic aneurysm model.
[Fig. 6] Fig. 6 shows graphs showing results of in vivo administration tests evaluating effects of antisense oligonucleotides on levels of AST (A), ALT (B), T-Bil (C) and CRE (D) in mice of an aortic aneurysm model.
[Fig. 7] Fig. 7 shows graphs showing results of in vitro administration tests evaluating effects of antisense oligonucleotides on expression levels of miR-33a (A), miR-33b (B) and ABCA1 mRNA (C) in human THP-1 cells.
[Fig. 8] Fig. 8 is a graph showing results of in vitro administration test evaluating effects of antisense oligonucleotides on a SREBF1 expression level in human THP-1 cells.
[Fig. 9] Fig. 9 shows graphs showing results of in vitro administration tests evaluating effects of antisense oligonucleotides on a MMP-9 mRNA expression level(A), a MCP-1 mRNA expression level (B), and a JNK1 mRNA expression level (C) in human THP-1 cells.
[Fig. 10] Fig. 10 shows graphs showing results of in vitro administration tests evaluating effects of antisense oligonucleotides on an enzyme activity level of pro-type MMP-9 protein (A), an enzyme activity level of truncated MMP-9 protein (B), an expression level of phosphorylated JNK1 protein (C), an expression level of JNK1 protein (D) and an expression level of phosphorylated JNK1 protein relative to a total JNK1 protein amount (E) in human THP-1 cells.

### [Embodiments for Carrying Out the Invention]

### (Definitions)

A "nucleic acid" refers to a molecule formed of nucleotide units. Examples of nucleic acids include natural nucleic acids (ribonucleic acid (RNA), and deoxyribonucleic acid (DNA)), and non-natural nucleic acids; forms of nucleic acids include single-stranded nucleic acids and double-stranded nucleic acids; and examples of functional nucleic acids include, but are not limited to, small interfering ribonucleic acid (siRNA), microRNA (miRNA), and the like. A nucleic acid can also include combinations of these components in a single molecule.

A "nucleobase" means a heterocyclic moiety that can pair with a base of another nucleic acid. Nucleobases include "modified nucleobases" and "unmodified nucleobases."

A "nucleoside" means a nucleobase linked to a sugar. In certain embodiments, a nucleoside is linked to a phosphate group.

A "nucleotide" means a nucleoside having a phosphate group or the like covalently linked to a sugar moiety of the nucleoside. A naturally occurring nucleotide has a ribose or deoxyribose sugar moiety and is covalently linked to a phosphate group by a phosphodiester bond to form an oligonucleotide or a polynucleotide.

A "modified nucleotide" means a nucleotide having, independently, a modified sugar moiety, a modified internucleoside linkage or a modified nucleobase. A "modified nucleoside" means a nucleoside having, independently, a modified sugar moiety or a modified nucleobase.

An "unmodified nucleotide" means a nucleotide formed of a naturally occurring nucleobase, a sugar moiety and an internucleoside linkage. In certain embodiments, an unmodified nucleotide is, but is not limited to, an RNA nucleotide (that is, a nucleotide having a β-D-ribonucleoside) or a DNA nucleotide (that is, a nucleotide having a β-D-deoxyribonucleoside).

An "internucleoside linkage" refers to a chemical bond between nucleosides.

A "modified internucleoside linkage" refers to a substitution or any change from a naturally occurring internucleoside linkage (that is, a phosphodiester internucleoside linkage). For example, there is a phosphorothioate internucleoside linkage, but it is not limited to this.

A "phosphorothioate internucleoside linkage" means an internucleoside linkage in which a phosphodiester linkage is modified by replacing one of non-bridging oxygen atoms with a sulfur atom. A phosphorothioate linkage is one example of the modified internucleoside linkage.

A "modified nucleobase" refers to any nucleobase other than adenine, cytosine, guanine, thymidine or uracil. For example, there is a 5-methylcytosine, but it is not limited to this. "Unmodified nucleobases" means the purine bases adenine (A) and guanine (G), and the pyrimidine bases thymine (T), cytosine (C) and uracil (U).

"miRNA" is a cleavage product of pre-miR by an enzyme Dicer, and means an endogenous non-coding RNA of a length of 18 to 25 nucleobases. Examples of miRNA can be confirmed in a miRNA database called miRBase (http://microrna.sanger.ac.uk/). In certain embodiments, miRNA is abbreviated as "miR" or "microRNA."

"pre-miR" is a cleavage product of a pri-miR by a double-stranded RNA-specific ribonuclease called Drosha, and means a non-coding RNA having a hairpin structure.

A "stem-loop sequence" means an RNA that has a hairpin structure and contains a mature miRNA sequence. In certain embodiments, a stem-loop sequence is a pre-miR. Examples of stem-loop sequences can be confirmed in a miRNA database called miRBase (http://www.mirbase.org/).

"pri-miR" is a substrate of a double-stranded RNA-specific ribonuclease Drosha, and means a non-coding RNA having a hairpin structure.

A "miRNA precursor" means a transcript containing a non-coding structured RNA that is derived from a genomic DNA and contains one or more miRNA sequences. For example, in certain embodiments, a miRNA precursor is a pre-RNA. In certain embodiments, a miRNA precursor is a pri-miR. In certain embodiments, a miRNA precursor is a stem-loop sequence.

A "target mRNA of miRNA" means a direct target mRNA of the miRNA. A miRNA is completely or nearly completely complementary to a target mRNA, and often complementarily binds to a seed sequence (a sequence of 7 bases of 2nd - 8th from a 5' end side of the miRNA), resulting in destabilization or translation inhibition of the target mRNA.

A "target protein of miRNA" means a protein produced by translation of a target mRNA of the miRNA.

A "miR-33b stem-loop sequence" means a pre-miR of a miRNA-33b having a nucleobase sequence

GCGGGCGGCCCGCGGUGCAUUGCUGUUGCAUUGCACGUGUGAGGCGGGUGCA
GUGCCUCGGCAGUGCAGCCCGGAGCCGGCCCCUGGCACCAC (SEQ ID NO: 4).

"miR-33a" means a miRNA having a nucleobase sequence GUGCAUUGUAGUUGCAUUGCA (SEQ ID NO: 2). (In the present specification, a base sequence is described in an order of from 5' to 3'.)

"miR-33b" means a miRNA having a nucleobase sequence GUGCAUUGCUGUUGCAUUGC (SEQ ID NO: 1).

"miR-33" means a miRNA having a nucleobase sequence GUGCAUUGUAGUUGCAUUGCA (SEQ ID NO: 3).

In humans, miR-33a and miR-33b are present. In mice, miR-33 is present. The human miR-33a and the mouse miR-33 have the same sequence. Therefore, in mice, only miRNA corresponding to the human miR-33a is present, and no miR corresponding to the human miR-33b is present.

A "miR-33b seed sequence (also referred to as a seed region)" refers to UGCAUUG in the miR-33b nucleobase sequence. SEQ ID NO: 1 or 2 includes two seed sequences.

A "miR-33a level," a "miR-33b level," and a "miR-33 level" respectively mean presences (levels), that is, expression levels, of miR-33a, miR-33b, and miR-33 that function as microRNAs in cells.

An "antisense oligonucleotide" is an antisense nucleic acid (RNA or DNA) that can hybridize to a target nucleic acid. In the present application, the term "antisense oligonucleotide" means a molecule that binds to a miRNA and inhibits activity or function of the miRNA. An antisense oligonucleotide in the present application may contain an artificial nucleic acid molecule in addition to a natural nucleic acid molecule.

"siRNA (small interfering RNA)" is a small double-stranded RNA formed of 15 - 30 base pairs. A siRNA is involved in a phenomenon called RNA interference, and suppresses expression of a nucleic acid in a sequence-specific manner by destruction of a target nucleic acid. In the present application, siRNA means a molecule functioning as an antagonist that destroys miR-33a and miR-33b and suppresses expression of miR-33a and miR-33b. siRNA in the present application may contain an artificial nucleic acid molecule in addition to a natural nucleic acid molecule.

"shRNA (short hairpin RNA)" has a hairpin structure and is subjected to processing to produce miRNA or siRNA (short interference RNA). In certain embodiments, shRNA is transcribed from a shRNA expression vector in cells.

"Complementary" means an ability with respect to pairing between nucleobases of a first nucleic acid and a second nucleic acid. Specifically, for example, adenine is complementary to thymidine or uracil, cytosine is complementary to guanine, and 5-methylcytosine is complementary to guanine. However, it is not limited to these.

"Fully complementary (also called complementarity)" or "100% complementary (also called complementarity)" means that all nucleobases in a nucleobase sequence of a first nucleic acid have complementary nucleobases in a second nucleobase sequence of a second nucleic acid. In certain embodiments, a first nucleic acid is a modified oligonucleotide and a target nucleic acid is a second nucleic acid.

"Hybridization" means annealing of complementary nucleic acid molecules.

"Mismatch" or "non-complementary nucleobase" refers to a case where a nucleobase of a first nucleic acid cannot pair with a corresponding nucleobase of a second nucleic acid or a target nucleic acid.

"Prevention or prophylactic" means delaying or preventing the onset or development of a disease, disorder, unfavorable health condition, or one or more symptoms associated with the disease, disorder or unfavorable health condition over a period from a few minutes to an indefinite period. Preventing also means reducing a risk of developing a disease, disorder, or unfavorable health condition.

"Treatment or therapy" means alleviating or eliminating a disease, disorder, or unfavorable health condition, or one or more symptoms associated with the disease, disorder, or unfavorable condition, or partially eliminating or eradicating one or more causes of the disease, disorder, or unfavorable health condition itself.

In the following, embodiments of the present invention are described in detail. The present invention is not limited to the following embodiments, and can be variously modified and implemented within the scope of its gist.

(miR-33b inhibiting substance)

The present invention is a therapeutic or prophylactic agent for aneurysms, which comprises a miR-33b inhibiting substance (hereinafter may be referred to as "the compound of the present invention"). The miR-33b inhibiting substance inhibits expression or function of miR-33b and, as a result, preferably suppresses an activity level of MMP-9, an expression level of MCP-1 and/or an expression level of phosphorylated JNK1 in cells involved in aneurysms, for example, monocytes or macrophage cells (hereinafter may be referred to as "cells").

The miR-33b inhibiting substance inhibits the expression or function of miR-33b, and as a result, preferably increases an intracellular presence level of a target mRNA of miR-33b and/or a target protein of miR-33b. An example of a target for miR-33b is an ATP-binding cassette transporter A1 (ABCA1). That is, a target molecule of miR-33b such as ABCA1 is normally subjected to expression inhibition by miR-33b, but an intracellular presence level thereof is increased due to that miR-33b is inhibited by the miR-33b inhibiting substance.

Specifically, an example of the miR-33b inhibiting substance is a substance that reduces a level of molecules that function as miR-33b in cells (hereinafter, this substance may be referred to as a "substance reducing miR-33b level"). Reduction in miR-33b level means reducing a level of miR-33b having a function that acts intracellularly on a target mRNA of miR-33b to reduce a level of the target mRNA and/or protein in cells through destabilization or translation inhibition of the mRNA.

In the present invention, a target mRNA of miR-33b may be anything as long as miR-33b directly binds to reduce an expression level thereof. However, specific examples thereof include ATP-binding cassette transporter A1 (ABCA1), carnitine palmitoyl transferase 1 (CPT1), and the like. Any one or more of these target mRNAs may be used.

Further, the substance reducing miR-33b level may be at least a substance that reduces a level of miR-33b in cells, and, for example, may further have an effect of reducing a level of miR-33a in the cells. However, in this case, preferably, it is preferably a substance of which an effect of reducing an intracellular level of miR-33b is stronger than an effect of reducing an intracellular level of miR-33a, and, in a system in which a miR-33b level in THP-1 cells (to be described later) is measured, a substance reducing miR-33b level which has a reducing rate of miR-33b level higher than a reducing rate of miR-33a level is preferably used. The reducing rate of miR-33b level can be calculated, for example, according to 100×((the miR-33b level when the substance reducing miR-33b level is not added) - (the miR-33b level when the substance reducing miR-33b level has been added))/(the miR-33b level when the substance reducing miR-33b level is not added). Further, the reducing rate of miR-33a level can be calculated, for example, according to 100×((the miR-33a level when the substance reducing miR-33b level is not added) - (the miR-33a level when the substance reducing miR-33b level has been added))/(the miR-33a level when the substance reducing miR-33b level is not added).

As a substance reducing miR-33b level which has a reducing rate of miR-33b level higher than a reducing rate of miR-33a level, specifically, for example, a value of (the reducing rate of miR-33a level)/(the reducing rate of miR-33b level) when calculated is 0.8 or less, 0.7 or less, 0.6 or less, 0.5 or less, 0.4 or less, 0.3 or less, 0.2 or less, or 0.1 or less, and is preferably 0.6 or less, more preferably 0.3 or less, and even more preferably 0.1 or less.

Reduction in miR-33b level in cells due to the substance reducing miR-33b level of the present invention means that, for example, the miR-33b level in the cells is reduced by causing the cells to be in contact with the substance reducing miR-33b level as compared to a case on non-contact or a case of being in contact with a negative control substance. Here, a degree of the reduction in miR-33b level in the cells may be any degree as long as it is a degree such that the miR-33b level in the cells is reduced and, as a result, a level of a target mRNA of miR-33b and/or a target protein of miR-33b (to be described later) in the cells is increased, or a degree such that the miR-33b level in the cells is reduced and, as a result, a phenotype of miR-33b-expressing cells leads to the adjustment of a disease-related substance to improve the disease. However, specifically, for example, after causing the cells to be in contact with the miR-33 level reducing substance of the present invention, it is preferable that the intracellular level of miR-33b is at least 50% or less, preferably 30% or less, and more preferably 10% or less, as compared to the case of non-contact or the case of being in contact with a negative control substance.

On the other hand, a degree of an increase in target mRNA expression level of miR-33b due to the substance reducing miR-33b level may be any degree as long as it is a degree such that a disease-related substance adjusted by a protein encoded by the target mRNA is adjusted in a direction of improving the disease. However, for example, it is a degree such that, after causing the cells to be in contact with the substance reducing miR-33b level, the target mRNA expression level is increased by at least 1.2 or more times, preferably 1.5 or more times, and even more preferably 2 or more times as compared to the case of non-contact or the case of being in contact with a negative control substance.

That a disease-related substance adjusted by a protein encoded by the target mRNA is adjusted in a direction of improving the disease is, specifically, for example, suppression of an inflammatory state of the cells, and, for example, it is that an mRNA expression level of MMP-9, MCP-1 and/or JNK1 or an expression level of a protein encoded by these is suppressed, that an MMP-9 activity is suppressed, and/or that phosphorylation of JNK1 is suppressed. A degree of the suppression of the mRNA expression level of MMP-9, MCP-1 and/or JNK1 or the expression level of a protein encoded by these due to the substance reducing miR-33b level is, for example, such that, after causing the cells (such as macrophage cells) to be in contact with the substance reducing miR-33b level, the mRNA expression level of MMP-9, MCP-1 and/or JNK1 or the expression level of a protein encoded by these is 0.8 or less, 0.7 or less, 0.6 or less, 0.5 or less, 0.4 or less, 0.3 or less, 0.2 or less or 0.1 or less, preferably 0.6 or less, more preferably 0.3 or less, and even more preferably 0.1 or less, as compared to the case of non-contact or the case of being in contact with a negative control substance. Further, a degree of the suppression of the MMP-9 activity and a degree of the suppression of the phosphorylation of JNK1 due to the substance reducing miR-33b level are such that, after causing the cells (such as macrophage cells) to be in contact with the substance reducing miR-33b level, the MMP-9 activity and/or the phosphorylation level of JNK1 are 0.8 or less, 0.7 or less, 0.6 or less, 0.5 or less, 0.4 or less, 0.3 or less, 0.2 or less or 0.1 or less, preferably 0.6 or less, more preferably 0.3 or less, and even more preferably 0.1 or less, as compared to the case of non-contact or the case of being in contact with a negative control substance.

Further, regarding an increase in expression level of a protein encoded by the target mRNA due to the compound of the present invention, it may be in any degree as long as it is a degree such that a disease-related substance adjusted by a target protein is adjusted in a direction of improving the disease. However, for example, it is a degree such that, after causing the cells to be in contact with the compound of the present invention, the expression level of target protein is increased by at least 1.2 or more times, preferably 1.5 or more times, and even more preferably 2 or more times as compared to the case of non-contact or the case of being in contact with a negative control substance.

The miR-33b inhibiting substance may be a substance that inhibits a function of miR-33b. That a function of miR-33b is inhibited by the compound of the present invention means that a disease-related substance is adjusted in a direction of improving the disease by increasing an expression level of a target mRNA, and/or a target protein of miR-33b, or by acting on a target nucleic acid of miR-33b and, as a result, suppressing a miR-33b-specific signaling pathway. That a disease-related substance is adjusted in a direction of improving the disease is, specifically, for example, suppression of an inflammatory state of the cells, and, for example, it is that an expression level of an mRNA or a protein of MMP-9, MCP-1 and/or JNK1 is suppressed, that an MMP-9 activity is suppressed, and/or that phosphorylation of JNK1 is suppressed. Therefore, the compound of the present invention can be used for suppressing the expression level of an mRNA of MMP-9, MCP-1 and/or JNK1. Further, the compound of the present invention can be used for suppressing the expression level of a protein of MMP-9, MCP-1 and/or JNK1.

Specific examples of the substance reducing miR-33b level include an antisense oligonucleotide, a siRNA, and an shRNA against miR-33b, or vectors expressing these. However, an antisense oligonucleotide against miR-33b (hereinafter may be referred to as "the antisense oligonucleotide of the present invention") is preferably used. Further, as the miR-33b inhibiting substance, a compound that inhibits a function of miR-33b may be used.

The antisense oligonucleotide of the present invention is an antisense oligonucleotide that reduces an intracellular level of miR-33b, and (1) is formed of 7 to 20 residues and (2) is 90% or more complementary to an equal length portion of a nucleobase sequence of the miR-33b stem-loop sequence described in SEQ ID NO: 4, preferably miR-33b (also referred to as hsa-miR-33b-5p) described in SEQ ID NO: 1. An equal length portion means a portion having complementarity between each nucleobase sequence of an antisense oligonucleotide and a nucleobase sequence of miR-33b in a specific base sequence. That is, a nucleobase sequence of the antisense oligonucleotide of the present invention is desirably fully complementary to an equal length portion of the nucleobase sequence of miR-33b, but may have one or more mismatched nucleobases, and has a complementarity of 85% or more, 90% or more, and preferably 95% or more. A nucleobase sequence of the compound of the present invention having such a mismatch may be any sequence as long as it can hybridize with miR-33b and reduce an intracellular level of miR-33b. However, in order to reduce binding to miR-33a, the nucleobase sequence preferably does not have a mismatch with 9th and 10th nucleobases counting from a 5' end of a nucleobase sequence of miR-33b described in SEQ ID NO: 1.

The above mismatched nucleobases may be clustered or may be sandwiched between complementary nucleobases, and do not need to be continuous with each other or with complementary nucleobases. Percent complementarity of the antisense oligonucleotide of the present invention with respect to miR-33b can be determined, for example, conventionally using the BLAST program (basic local alignment search tools) and the PowerBLAST program which are known in the art (Altschul et al., J. Mol. Biol., 1990, 215, 403 410; Zhang and Madden, Genome Res., 1997, 7, 649 656). Percent homology, sequence identity or complementarity, can be determined, for example, by the Gap program (Wisconsin Sequence Analysis Package, Version 8 for Unix, Genetics Computer Group, University Research Park, Madison Wis.), using default settings using the algorithm of Smith and Waterman (Adv. Appl. Math., 1981, 2, 482 489).

The nucleobase sequence of miR-33b described in SEQ ID NO: 1 is contained in the miR-33b stem-loop sequence, and the miR-33b nucleobase sequence targeted by the compound of the present invention also includes the full length of the miR-33b stem-loop sequence.

The nucleobase sequence of the antisense oligonucleotide of the present invention may be any sequence as an equal length portion of a complementary strand of a base sequence of the miR-33b stem-loop sequence described in SEQ ID NO: 4, preferably a complementary strand of a miR-33b base sequence of SEQ ID NO: 1, and may have any sequence length, for example, may have a sequence length of 7, 8, 9, 10, 11, 12, 13, 14, 15, 16, 17, 18, 19 or 20 residues, and preferably contains a sequence complementary to the 9th - 10th counting from the 5' end in the nucleobase sequence of miR-33b described in SEQ ID NO: 1, and the sequence length is preferably 12 - 19 residues, and more preferably 12 - 16 residues.

### Specific examples thereof include

AACAGCAATGCA (SEQ ID NO: 5) or
TGCAACAGCAATGCAC (SEQ ID NO: 6), or the like, and
AACAGCAATGCA (SEQ ID NO: 5) is preferably used.

In certain embodiments, it is an antisense oligonucleotide in which a portion of a, or an entire, cytosine base of a sequence of SEQ ID NO: 5 or 6 is a 5-methylcytosine. Examples of a modified oligonucleotide in which a portion of a cytosine base is a 5-methylcytosine include SEQ ID NOs: 8 and 10.

The antisense oligonucleotide of the present invention can be a modified oligonucleotide. The modified oligonucleotide may contain a modified base such as a 5-methylcytosine described above, or at least one nucleoside forming the oligonucleotide may contain a modified sugar.

In the present invention, a modified sugar refers to a sugar in which a sugar moiety is modified, and a modified oligonucleotide containing one or more of such modified sugars has advantageous features such as an enhanced nuclease stability and an increased binding affinity. Preferably, at least one of the modified sugars has a bicyclic sugar or a substituted sugar moiety.

Examples of nucleosides having a modified sugar include nucleosides containing 5'-vinyl, 5'-methyl (R or S), 4'-S, 2'-F, 2'-OCH₃, 2'-OCH₂CH₃, 2'-OCH₂CH₂F and 2'-O(CH₂)₂OCH₃ substituents. The substituent at the 2' position can also be selected from allyl, amino, azido, thio, O-allyl, O-C₁ - C₁₀ alkyl, OCF₃, OCH₂F, O(CH₂)₂SCH₃, O(CH₂)₂-O-N(Rₘ)(Rₙ), O-CH₂-C(=O)-N(Rₘ) (Rₙ) and O-CH₂-C(=O)-N(R₁)-(CH₂)₂-N(Rₘ)(Rₙ) (wherein R₁, Rₘ and Rₙ are each independently H or substituted or unsubstituted C₁ - C₁₀ alkyl).

Examples of nucleosides having a bicyclic sugar include nucleosides that contain a bridge between the 4' and the 2' ribosyl ring atoms. In certain embodiments, an oligonucleotide provided herein includes a nucleoside having one or more bicyclic sugars, in which a bridge includes one of the following formulas: 4'-(CH₂)-O-2' (LNA); 4'-(CH₂)-S-2'; 4'-(CH₂)₂-O-2' (ENA); 4'-CH(CH₃)-O-2' and 4'-CH(CH₂OCH₃)-O-2' (and analogs thereof, see U.S. Patent No. 7,399,845); 4'-C(CH₃)(CH₃)-O-2' (and analogs thereof, see WO 2009/006478); 4'-CH₂-N(OCH₃)-2' (and analogs thereof; see WO 2008/150729); 4'-CH₂-O-N(CH₃)-2' (see US 2004-0171570); 4'-CH₂-N(R)-O-2' (wherein R is H, C₁ - C₁₂ alkyl or a protecting group) (see U.S. Patent No. 7,427,672); 4'-CH₂-C(H)(CH₃)-2' (see Chattopadhyaya et al., J. Org. Chem., 2009, 74, 118 - 134); and 4'-CH₂-C(=CH₂)-2' (and analogues thereof; see WO 2008/154401).

In certain embodiments (AmNA), examples of a nucleoside having a bicyclic sugar include nucleosides represented by the following formula: 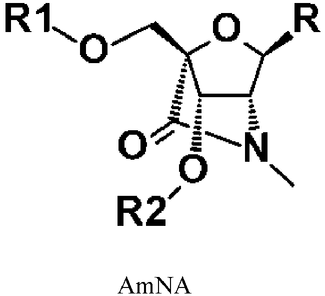 [wherein, R is a nucleobase, and R1 and R2 each independently indicate a phosphate group that may be substituted].

A method for preparing a modified sugar is well known to a person skilled in the art by WO11/052436 and the like.

In certain embodiments, the antisense oligonucleotide of the present invention has a nucleobase sequence in which at least one nucleobase is a cytosine. In certain embodiments, at least one cytosine is a 5-methylcytosine of a modified nucleobase. In certain embodiments, all cytosines are 5-methylcytosines.

A naturally occurring intemucleoside linkage of RNA and DNA is a 3'-5' phosphodiester linkage. An oligonucleotide having one or more modified, that is, non-naturally occurring, intemucleoside linkages is often preferred over an oligonucleotide having a naturally occurring intemucleoside linkage because of properties such as, for example, enhanced cellular uptake, enhanced affinity for a target nucleic acid, and increased stability in the presence of nucleases.

An oligonucleotide having modified internucleoside linkages include internucleoside linkages that retain a phosphorus atom and internucleoside linkages that do not have a phosphorus atom. Representative phosphorus containing internucleoside linkages include, but are not limited to, one of more of phosphodiesters, phosphotriesters, methylphosphonates, phosphoramidate, and phosphorothioates. Methods for preparing phosphorous-containing and non-phosphorous-containing linkages are well known.

In certain embodiments, internucleoside linkages of the modified oligonucleotide of the present invention are all phosphorothioate internucleoside linkages.

In the present specification, in symbols such as "Gas" representing nucleotides, an abbreviation shown at a left position means a nucleobase portion, an abbreviation shown at a center position means a sugar moiety, and an abbreviation shown at a right position means a mode of an internucleoside linkage.

In certain embodiments, the antisense oligonucleotide is a modified oligonucleotide represented by the following formula:

Tas Gds Mas Ads Aas Cds Aas Gds Mas Ads Aas Tds Gas Cds Aas Cd,

wherein nucleobases are represented according to the following symbols:
A = adenine, T = thymine, G = guanine, C = cytosine, M = 5-methylcytosine,
sugar moieties are represented according to the following symbols:
   a = a sugar moiety of AmNA, d = 2'-deoxyribose,
   and internucleoside linkages are represented according to the following symbol:
      s = phosphorothioate.

In certain embodiments, the antisense oligonucleotide is a modified oligonucleotide represented by the following formula:

Aas Ads Mas Ads Gas Cds Aas Ads Tas Gds Mas Ad,

wherein nucleobases are represented according to the following symbols:
A = adenine, T = thymine, G = guanine, C = cytosine,
sugar moieties are represented according to the following symbols:
   a = a sugar moiety of AmNA, d = 2'-deoxyribose,
   and internucleoside linkages are represented according to the following symbol:
      s = phosphorothioate.

### (Method for selecting a miR-33b inhibiting substance)

A method for selecting the compound of the present invention may be any method as long as it is a method that can verify that an intracellular level of miR-33b is reduced or a function of miR-33b is inhibited by the compound of the present invention. However, in the compound of the present invention, since a substance reducing miR-33b level which has a reducing rate of miR-33b level higher than a reducing rate of miR-33a level is preferably used, a method that can verify reduction of intracellular levels of miR-33b and miR-33a is preferably used. Specifically, for example, the following in vitro and in vivo verification methods are used.

The in vitro verification with respect to reduction of an intracellular level of miR-33b of the compound of the present invention can use any cell line as long as it is a cell line that expresses miR-33b, preferably, a cell line that expresses miR-33b and mirR-33a (hereinafter, it may be referred to a "miR-33b expression cell line"). However, for example, THP-1 cells (human macrophage cells, for example, ATCC TIB-202) can be used. Further, it is also possible to select a cell line into which human miR-33b, preferably miR-33b and miR-33a, has been introduced. A cell line used for introducing miR-33b, or miR-33b and miR-33a, is not particularly limited as long as it is a cell that is derived from an animal and is normally used. These cell lines are available from commercial suppliers and are cultured using commonly used commercially available reagents and according to supplier's instructions. Generally, examples of methods for introducing miR-33b, or miR-33b and miR-33a, into these cell lines include, but are not limited to, a method in which a vector for expression of miR-33b, or miR-33b and miR-33a, is transfected, and the like.

A method for causing the compound of the present invention to be in contact with a miR-33b expression cell line is also not particularly limited. However, an example thereof is a method that is generally used for introducing a nucleic acid into cells. Specific examples thereof include a lipofection method, an electroporation method, a Gymnosis method, and the like.

An intracellular level of miR-33b or miR-33a can be assayed using various methods known in the art. Specific examples include Northern blot analysis, competitive polymerase chain reaction (PCR) or quantitative real-time PCR, and the like. When isolating miRNAs, a method commonly known in the art is used, for example, TriPure Isolation Reagent (Roche), Maxwell RSC miRNA Tissue Kit Reagent (Promega), and the like can be used according to manufacturers' recommended protocols. In this way, an expression level of miR-33b, or miR-33b and miR-33a, can be analyzed.

Tests can be conducted in vivo to evaluate an ability of the compound of the present invention to reduce an intracellular level of miR-33b or to change a phenotype of miR-33b expression cells. For the verification of the reduction of the intracellular level of miR-33b of the compound of the present invention, for example, a method can be used in which the compound of the present invention is administered to an animal expressing miR-33b, preferably miR-33b and miR-33a, and the above-described analysis of an expression level of miR-33b or miR-33b and miR-33a in the cells is performed. Further, the evaluation of the ability of the compound of the present invention to change the phenotype of the miR-33b expression cells can be carried out using an experimental disease model, such as an aneurysm model, such as a calcium chloride coating model.

The in vitro or in vivo verification with respect to the miR-33b level reduction or the miR-33b function inhibition of the compound of the present invention can be performed by administering the compound of the present invention to an animal that expresses a miR-33b expression cell line or miR-33b, preferably miR-33b and miR-33a, and measuring an expression level of mRNA or a protein of MMP-9, MCP-1 and/or JNK1.

### (Method for producing the antisense oligonucleotide of the present invention)

The antisense oligonucleotide of the present invention can be synthesized using a conventional method, for example, it can be easily synthesized using a commercially available nucleic acid synthesizer. Further, a sugar-modified AmNA of a nucleoside, which may be contained in the antisense oligonucleotide, can be synthesized using a method disclosed in WO11/052436.

The siRNA and shRNA of the present invention can be artificially chemically synthesized. Further, siRNA and shRNA can be synthesized as antisense and sense RNA from template DNA in vitro using, for example, T7 RNA polymerase and T7 promoter.

A compound having an activity of inhibiting miR-33b in the present invention can be synthesized, for example, using methods disclosed in Proc. Natl. Acad. Sci.USA, 113 (21): 5898-903 (2016); Mol. Pharm.,16 (2): 914-920 (2019); J. Am. Chem. Soc., 139 (9): 3446-3455 (2017); and the like.

### (Treatment of aneurysms using the compound of the present invention)

Aneurysms can be treated or prevented using the miR-33b inhibiting substance in the present invention.

Maim pathological conditions of aneurysms are chronic inflammation and extracellular matrix metabolism disorder due to increased inflammatory mediator and the like. These are interrelated and amplify and prolong pathological conditions, and cause aortic aneurysms to progress.

Known examples of inflammatory mediators include: inflammatory cytokines such as chemoattractant protein (MCP-1), tumor necrosis factor (TNF), interleukin (IL)-6, and IL-1; lipid mediators such as prostaglandin and leukotriene; and nitric oxide or various oxidative stresses of gas mediators. It is thought that inflammatory mediators induce various inflammatory cells, and infiltrated inflammatory cells secrete additional inflammatory mediators to amplify and prolong a pathological process of inflammation.

Further, inflammatory mediators activate intracellular signal transduction molecules. Examples of intracellular signal transduction molecules include Janus kinase (JAK), mitogen activated protein (MAP) kinase, nuclear factor (NF)-xB, signal transducer and activator of transcription (STAT), transforming growth factor (TGF), SMAD, and the like.

Activation of signal transduction molecules promotes secretion of extracellular matrix degrading enzymes. As a result, degradation of elastin, collagen, and the like, which are main constituents of the aortic wall, is accelerated, causing weakening of the aortic wall. Examples of extracellular matrix degrading enzymes include matrix metalloproteinase (MMP)-9 or MMP-2. Further, activation of signal transduction molecules suppresses production of extracellular matrix synthesizing enzymes and induces smooth muscle cell death.

In this way, as an extracellular matrix metabolic balance tends to increase degradation, smooth muscle cells are reduced and the wall is weakened, leading to formation and progression of aneurysms. Such production of inflammatory mediators and activation of signal transduction molecules occur in cells involved in aneurysms, such as macrophages, monocytes, vascular endothelial cells or smooth muscle cells. For example, suppressing macrophage activation is important to prevent the formation and progression of aneurysms.

As a result of studies conducted by the present inventors, as shown in Example 2 to be described later, the miR-33b inhibiting substance in the present invention reduced an intracellular level of miR-33b and suppressed formation and progression of aneurysms. As shown in Example 2 to be described later, examples of mechanisms for suppressing formation and progression of aneurysms by a miR-33b inhibiting substance include suppression of expression of inflammatory mediator MCP-1, suppression of activation of intracellular signal transduction molecules JAK and/or suppression of activation of extracellular matrix degrading enzymes MMP-9. However, since there are many miR-33b downstream targets and there are multiple signal transduction pathways, the mechanisms are not limited to these. On the other hand, as shown in Example 2 to be described later, an antisense oligonucleotide against miR-33a increased expression of miR-33b and caused formation and progression of aneurysms.

Thus, in the present invention, the role of miR-33b in the formation of aneurysms is clarified for the first time, and it is found that aneurysms can be prevented or treated by using an inhibiting substance that selectively inhibits miR-33b.

As described above, since a miR-33b inhibiting substance can suppress development or expansion of aneurysms and can regress aneurysms, aneurysms can be treated, prevented, or alleviated.

By preventing weakening of the vessel wall, a miR-33b inhibiting substance can prevent development of aneurysms, can prevent further expansion of aneurysms, or can regress or eliminate aneurysms, and thereby, can prevent rupture of aneurysms.

In the present invention, aneurysms include all pathological conditions that are usually called aneurysms. Aneurysms are classified in various ways according to their site of occurrence, their cause, or their shape. Examples of classification according to the site of occurrence include: aortic aneurysms including thoracic aortic aneurysms and abdominal aortic aneurysms; visceral aneurysms such as cerebral aneurysms and renal aneurysms; aneurysms occurring in peripheral arteries; and the like. In the present invention, aortic aneurysm, cerebral aneurysms or renal aneurysms are preferable examples, and cerebral aneurysms or aortic aneurysms are more preferable examples, and aortic aneurysms are further more preferable examples. Aneurysms can be classified according to their wall structure into, for example, true aneurysms, dissecting aneurysms, pseudoaneurysms, and the like. Examples of classification according to the cause can include: atherosclerotic aneurysms; inflammatory aneurysms; congenital aneurysms; traumatic aneurysms; infectious aneurysms typified by bacterial aneurysms, fungal aneurysms, and syphilitic aneurysms; and the like. Examples of classification according to the shape can include saccular aneurysms, fusiform aneurysms, and the like. The present invention is not particularly limited to these classifications.

Aneurysms may be, for example, aneurysms associated with Marfan syndrome, porencephaly, Lois Dietz syndrome, autosomal dominant cerebral arteropathy, Ehlers-Danlos syndrome, familial aortic aneurysm dissection, hereditary cerebral microvascular disease, Kawasaki disease, Behcet's disease, or Takayasu's arteritis and thoracic aortic aneurysms, abdominal aortic aneurysm, cerebral aneurysms or renal aneurysms associated with these syndromes or diseases, but are not limited to these.

The miR-33b inhibiting substance in the present invention suppresses an expression level of mRNA or protein of MMP-9, MCP-1 and/or JNK1 in cells involved in aneurysms, such as macrophages, monocytes, vascular endothelial cells, or smooth muscle cells, and suppresses activation of MMP-9, and/or suppresses phosphorylation of JNK1. Therefore, it can be used for prevention and treatment of angiopathy diseases in which expression or activation of at least one of these factors is increased.

As described above, a miR-33b inhibiting substance can be used to treat or prevent aneurysms. Therefore, the present invention provides a miR-33b inhibiting substance for use in treatment of aneurysms; a pharmacological composition for use in treatment of aneurysms; use of a miR-33b inhibiting substance for treating aneurysms; use of a miR-33b inhibiting substance in manufacture of therapeutic agents for aneurysms; a miR-33b inhibiting substance for use in manufacture of therapeutic agents for aneurysms; and a method for treating or preventing aneurysms, the method comprising administering an effective amount of a miR-33b inhibiting substance to a subject in need thereof.

The composition of the present invention comprising the compound of the present invention or a pharmaceutically acceptable salt thereof and a pharmaceutically acceptable carrier can be used as a pharmaceutical composition. For preparation of the pharmaceutical composition, the antisense oligonucleotide can be mixed with one or more pharmaceutically acceptable active or inactive substances. A composition and a method for formulating a pharmaceutical composition can be selected according to several criteria including a route of administration, an extent of a disease or a dose to be administered.

For example, as a composition for parenteral administration, for example, an injection is used. Injections include dosage forms such as intravenous injections, subcutaneous injections, intradermal injections, intramuscular injections, drip injections, and intra-articular injections. Such injections are prepared according to methods commonly known per se, for example, by dissolving, suspending or emulsifying the antisense oligonucleotide in a sterile aqueous or oily solution usually used for injections. As an aqueous solution for injection, for example, phosphate buffered saline, physiological saline, an isotonic solution containing glucose or other adjuvants, and the like are used, and may be used in combination with a suitable solubilizing agent, for example, alcohol (for example, ethanol), polyalcohol (for example, propylene glycol, polyethylene glycol), nonionic surfactant [for example, polysolvate 80, HCO-50 (polyoxyethylene (50 mo1) adduct of hydrogenated castor oil)], and the like. Further, a buffering agent, a pH adjusting agent, an isotonizing agent, a soothing agent, a preservative, a stabilizer, and the like can be contained.

Examples of compositions for oral administration include solid or liquid dosage forms, specifically, tablets (including sugar-coated tablets and film-coated tablets), pills, granules, powders, capsules (including soft capsules), syrups, emulsions, suspensions, and the like. Such compositions are produced using methods commonly known per se and contain carriers, diluents or excipients commonly used in the pharmaceutical field. For example, as carriers and excipients for tablets, lactose, starch, sucrose, magnesium stearate, and the like are used.

Further, the pharmacological composition of the present invention can include a nucleic acid introduction reagent. As the nucleic acid introduction reagent, liposome, lipofectin, lipofectamine, DOGS (transfectum), DOPE, DOTAP, DDAB, DHDEAB, HDEAB, polybrene, or cationic lipids such as poly (ethyleneimine) (PEI), or the like can be used.

Further, the antisense oligonucleotide contained in the pharmaceutical composition of the present invention is preferably conjugated with a conjugate group such as cholesterol, sugar, phospholipid, biotin, phenazine, vitamin, peptide, folic acid, phenanthridine, anthraquinone, acridine, fluorescein, rhodamine, coumarin and a dye at one or more sites. The antisense oligonucleotide can be selected to enhance its activity, and its uptake into tissues or cells targeting an aneurysm site. The conjugate group is preferably cholesterol or lipid.

The conjugate group either directly binds to the antisense oligonucleotide, or the conjugate group binds to the antisense oligonucleotide by a linking moiety selected from amino, hydroxy, carboxylic acid, thiol, unsaturated moiety (for example, double or triple bond), 8-amino-3,6-dioxaoctanoic acid (ADO), succinimidyl 4-(N-maleimidemethyl) cyclohexane-1-carboxylate (SMCC), 6-aminocaproic acid (AHEX or AHA), substituted C₁ - C₁₀ alkyl, substituted or unsubstituted C₂ - C₁₀ alkenyl, and substituted or unsubstituted C₂ - C₁₀ alkynyl. Here, a substituent is selected from amino, alkoxy, carboxy, benzyl, phenyl, nitro, thiol, thioalkoxy, halogen, alkyl, aryl, alkenyl and alkynyl.

For the pharmaceutical composition of the present invention, a composition with few side effects can be selected. Side effects include injection site reactions, liver function test abnormalities, renal function abnormalities, liver toxicity, renal toxicity, central nervous system abnormalities, myopathies, malaise, and the like. For example, an elevated level of ALT, AST, or γ-GTP in the blood can indicate liver toxicity or liver function abnormality. For example, elevated bilirubin can indicate liver toxicity or liver function abnormality. Further, elevated urinary protein and elevated creatinine or BUN in the blood can indicate renal toxicity or renal function abnormality.

The pharmaceutical composition of the present invention can treat, prevent, or alleviate aneurysms by administering it to target individuals using an appropriate method. That is, the present invention provides a method for treating, preventing, or alleviating aneurysms by administering an effective amount of a miR-33b inhibiting substance, preferably an antisense oligonucleotide against miR-33b, or a pharmacological composition containing the antisense oligonucleotide, to target individuals in need thereof using an appropriate method.

A dosage form of the pharmaceutical composition of the present invention may be a normal systemic administration such as intravenous or intraarterial administration, or a local administration such as local injection or oral administration. Dosage of the pharmaceutical composition of the present invention may be changed as appropriate depending on a purpose of use, severity of the disease, age, body weight, gender, and the like of a patient. However, an amount of the antisense oligonucleotide can usually be selected from a range of 0.1 ng - 100 mg/kg/day, preferably 1 ng - 10 mg/kg/day.

### [Examples]

### Non-limiting disclosure and incorporation by reference

While certain compounds, compositions and methods described herein have been described with specificity in accordance with certain embodiments, the following examples serve only to illustrate the compounds described herein and are not intended to limit the same. Each of the references described in this application is incorporated herein by reference in its entirety.

### Example 1

### Synthesis and purification of antisense oligonucleotides

AmNA amidite was obtained from Osaka Synthetic Chemical Laboratories, Inc., and AmNA-containing antisense oligonucleotides were synthesized and purified by Ajinomoto Bio-pharma Services and GeneDesign, Inc.

The synthesized antisense oligonucleotides are shown in Table 1 below. 33b-2-AmNA and 33b-1-AmNA indicate oligonucleotides against miR-33b; 33a-2-AmNA and 33a-1-AmNA indicate oligonucleotides against miR-33a; and NEG-AmNA indicates a control antisense oligonucleotide. In the notation of the antisense oligonucleotides, each nucleotide is represented by three letters. However, a 3'-end nucleotide is represented by two letters since there is no internucleoside linkage.
1) The first letter is capitalized and indicates the following nucleobases:
   A = adenine, T = thymine, G = guanine, C = cytosine, M = 5-methylcytosine.
2) The second letter indicates the following sugar moieties:
   a = a sugar moiety of AmNA, d = 2'-deoxyribose.
3) The third letter indicates the following internucleoside linkage:
   s = phosphorothioate.

**[Table 1]**

| Name of antisense oligonucleotide | SEQ ID NO | Sequence | m/z |
|---|---|---|---|
| 33a-2-AmNA (12) | 7 | AasAdsMasTdsAasCdsAasAdsTasGdsMasAd | 4156.5 |
| 33b-2-AmNA (12) | 8 | AasAdsMasAdsGasCdsAasAdsTasGdsMasAd | 4181.33 |
| 33a-1-AmNA (16) | 9 | TasGdsMasAdsAasCdsTasAdsMasAdsAasTdsGasCdsAasCd | 5541.23 |
| 33b-1-AmNA (16) | 10 | TasGdsMasAdsAasCdsAasGdsMasAdsAasTdsGasCdsAasCd | 5566.28 |
| NEG-AmNA (12) | 11 | AasAdsMasAdsAasTdsAasCdsTasAdsMasGd | 4156.15 |

### Example 2

### Measurement of pharmacological effects of 33a-2-AmNA (12) and 33b-2-AmNA (12) in calcium chloride-induced aortic aneurysm model using miR-33b knock-in mice

MiR-33b knock-in mice with C57BL/6 as a background were used. As the miR-33b knock-in mice, those reported in SCIENTIFIC REPORTS 4:5312 DOI: 10.1038/srep05312 were used. An abdominal aortic aneurysm model coated with calcium chloride using 8-week-old miR-33b knock-in male mice (according to a method reported in Atherosclerosis 2013, 226: 29-39) was prepared. A method for anesthetizing the mice was to intraperitoneally administer a 3-type mixed anesthesia (medetomidine 0.3 mg/kg, midazolam 4.0 mg/kg, and butorphanol 1.0 mg/kg). In the abdominal aortic aneurysm model, separation from a subrenal artery to a common iliac artery bifurcation was performed, 0.5 mol/L calcium chloride soaked in gauze was applied to an outer surface thereof, the calcium chloride application gauze was removed after 20 minutes, and an area around an abdominal aorta was washed with gauze soaked with physiological saline and then the separation wound was sutured. One week or six weeks after an abdominal aortic aneurysm was created, a maximum diameter and a longitudinal extension distance of the abdominal aortic aneurysm were measured and analyzed. In this case, euthanasia was performed using 5 times a usual dose of the above-described 3-type mixed anesthesia, and PBS was refluxed from a left ventricle under a physiological pressure. In order to prevent collapse of a vascular structure externally, the maximum diameter and the longitudinal extension distance of the abdominal aortic aneurysm were measured using a measure before an aorta was removed from a body, and analyzed after photography.

In a group for which analysis was performed one week later, the antisense oligonucleotides (33a-2-AmNA (12), 33b-2-AmNA (12)) were subcutaneously injected at 10 mg/kg the day before a surgical procedure, the day after the surgical procedure, and 4 days after the surgical procedure. In a group for which analysis was performed six weeks later, subcutaneous injection at 10 mg/kg was performed the day before a surgical procedure, the day after the surgical procedure, and once a week from 1 week to 6 weeks after the surgical procedure. Seven days after a final administration, data was collected and various measurements were performed.

All mice were bred at Laboratory Animals Facility, School of Medicine, Kyoto University, and the facility was maintained in an SPF (specific pathogen free) state, and this study was approved by the Kyoto University Medical Ethics Committee.

Expression levels of miR-33a and miR-33b due to administration of the antisense oligonucleotides (NEG-AmNA (12), 33a-2-AmNA (12), 33b-2-AmNA (12)) were measured (hereinafter, in the figures, NEG-AmNA (12) may be referred to as "control," 33a-2-AmNA (12) may be referred to as "Anti-miR-33a," and 33b-2-AmNA (12) may be referred to as "Anti-miR-33b"). For miRNA measurement, RNA was separated and purified using TriPure Isolation Reagent (Roche), and was measured using TaqMan MicroRNA assay protocols (Applied Biosystems). A product was analyzed using a thermal cycler (ABI Prism 7900HT sequence detection system), and expression was standardized using U6 snRNA.

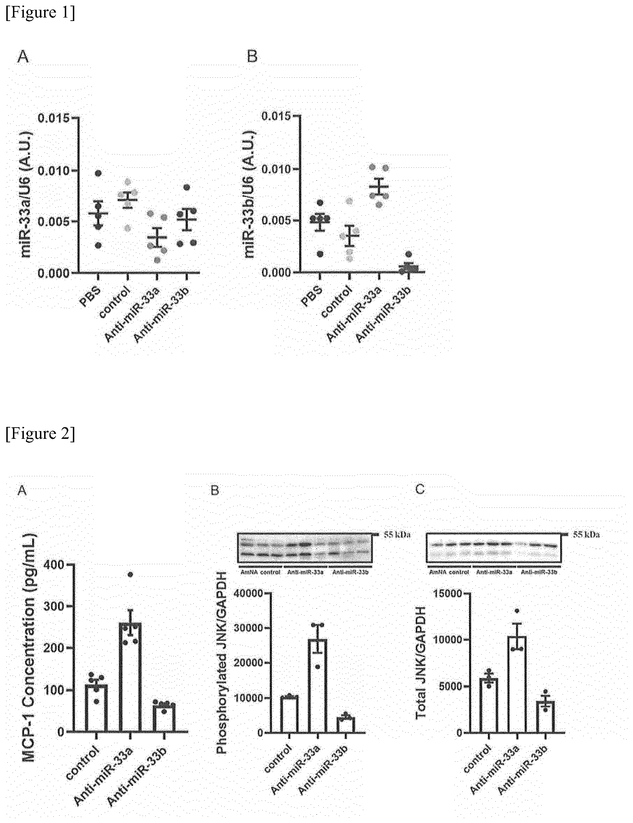

As shown in Fig. 1, in the group for which the analysis was performed after 6 weeks, in the aorta, strong suppression of miR-33a levels was observed in a 33a-2-AmNA (12) administration group, and strong suppression of miR-33b levels was observed in a 33b-2-AmNA (12) administration group. On the other hand, an increase in miR-33b level was observed in the 33a-2-AmNA (12) administration group. A value of (reducing rate of miR-33a)/(reducing rate of miR-33b level) was 0.23.

In a calcium chloride-induced aortic aneurysm model using miR-33b knock-in mice, one week after the induction, expression levels or phosphorylation levels of MMP-9, MCP-1 and JNK1 proteins due to administration of 33a-2-AmNA (12) and 33b-2-AmNA (12) were measured using the following method. A sufficiently anesthetized mouse was refluxed from the left ventricle using PBS at physiological pressure, and a sample was collected. Western blotting was performed using a standard method. A lysis buffer was used which was prepared with a complete mini protease inhibitor (Roche), ALLN (25 mg/mL), 0.5 mM NaF, and 10 mM Na3VO4, together with 100 mmol/L Tris-HCl, 75 mmol/L NaCl, and 1% Triton X-100. A protein concentration was measured using a bicinchoninic acid protein assay kit (Bio-Rad), and, after electrophoresis using a NuPAGE 4% -12% Bis-Tris (Invitrogen) gel, it was transferred to a Protran nitrocellulose transfer membrane (Whatman). The membrane was blocked using 1 - 5% non-fat milk and stirred overnight at 4 °C using Anti-phospho-SAPK/JNK (1: 500, Cell Signaling 4668S), anti-SAPK/JNK (1: 500, Cell Signaling 9252S), and Anti-GAPDH (1: 3000, Cell Signaling 2118S) as primary antibodies. After washing with PBS-0.05% Tween 20 (0.05% T-PBS), it was stirred at 4 °C for 1 hour using a secondary antibody (anti-rabbit, anti-mouse IgG-linked; 1:2000), after washing with PBS-0.05% Tween 20 (0.05% T-PBS), ECL Western Blotting Detection Reagent (GE Healthcare) was added for imaging.

In a gelatin zymography method, similar to the above, protein was extracted using a lysis buffer and 10 µg of protein was used for a measurement. The MMP-9 activity was measured using a Gelatin-Zymography Kit (Cosmo Bio) according to a package insert.

For the expression level of MCP-1 protein, 50 µL of a serum was collected as a sample after an abdominal aortic aneurysm model was prepared, and an MCP-1 concentration measurement was performed using an enzyme-linked immunosorbent assay (ELISA) kit (R&D Systems) according to a package insert.

As shown in Fig. 2, in the 33a-2-AmNA (12) administration group, an increase in the protein expression level of MCP-1 and JNK1 and an increase in the phosphorylation level of JNK1 were observed. On the other hand, in the 33b-2-AmNA (12) administration group, a decrease in the protein expression level of MCP-1 and JNK1 and a decrease in the phosphorylation level of JNK1 were observed.

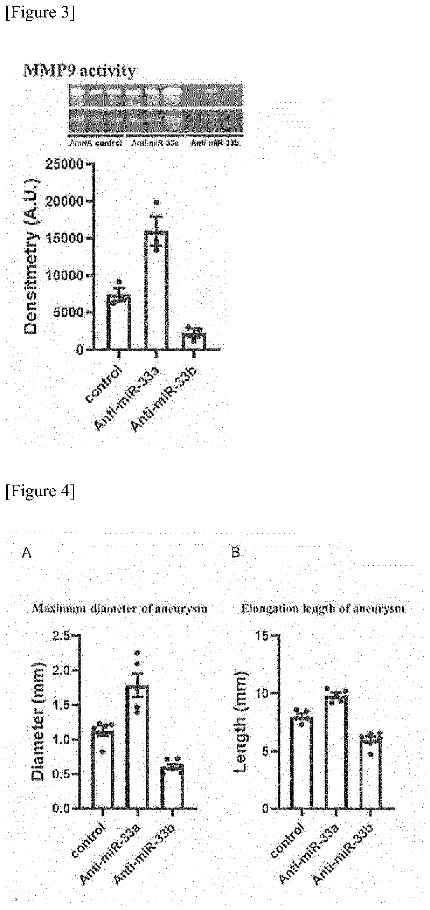

As shown in Fig. 3, an increase in the MMP-9 activity was observed in the 33a-2-AmNA (12) administration group, and a decrease in the MMP-9 activity was observed in the 33b-2-AmNA (12) administration group.

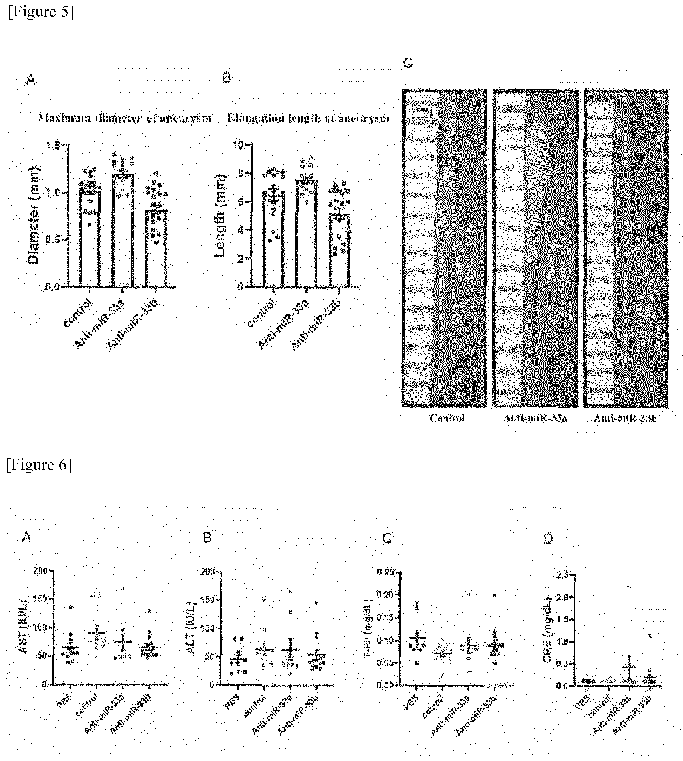

Next, influence of 33a-2-AmNA (12) or 33b-2-AmNA (12) on aneurysm formation was investigated using a calcium chloride-induced aortic aneurysm model using miR-33b knock-in mice. As a result, in an analysis after one week, as shown in Fig. 4, in 33b-2-AmNA (12), arterial diameter enlargement and arterial length extension that were observed in the control group (NEG-AmNA (12)) were not observed. Also in an analysis after six weeks, as shown in Fig. 5, in 33b-2-AmNA (12), arterial diameter enlargement and arterial length extension that were observed in the control group (NEG-AmNA (12)) were not observed. That is, an effect of suppressing aneurysm formation was observed. On the other hand, in 33a-2-AmNA (12), as compared to the control group, further enlargement of the arterial diameter and extension of the arterial length were observed, and aneurysm formation was promoted.

For a serum biochemical analysis, blood was collected from an inferior vena cava of an anesthetized mouse, and a serum was separated by centrifugation at 4 °C and was stored at - 80 °C. The biochemical analysis was performed using a Hitachi 7180 automatic analyzer (Nagahama Life Science). As a result, as shown in Fig. 6, no toxically serious findings were observed in AST, ALT, and T-BiI, which are liver function parameters, or in CRE, which is a renal function parameter.

### Example 3

### Measurement of pharmacological effects of 33a-2-AmNA (12) and 33b-2-AmNA (12) using human macrophage THP-1 cells

THP-1 cells were purchased from the American Type Cell Collection, were cultured using RPMI 1640 with 10% FBS added thereto, and were differentiated into THP-1 macrophages using 100 nM PMA (Nacalai Tesque). When AmNA was transfected to THP-1 macrophages, an experiment was performed using Lipofectamine RNAiMAX Transfection Reagent (Thermo Fisher SCIENTIFIC) according to a package insert. The AmNA-containing antisense oligonucleotides (NEG-AmNA (12), 33a-2-AmNA (12), 33b-2-AmNA (12)) were introduced at 50 nM, and cells were collected 4 days after the introduction, and RNA extraction and various RNA measurements were performed as follows.

RNA extraction and real-time PCR were performed according to the following procedure. RNA was separated and purified using TriPure Isolation Reagent (Roche), and, after cDNA was synthesized using a Transcriptor First Strand cDNA Synthesis Kit (Roche) according to a package insert, specific genes were amplified for 40 cycles using SYBRTM Green PCR Master Mix (Applied Biosystems). Expression was standardized using β-actin or U6 as a housekeeping gene. Sequences of primers used are as follows.
SREBF1 Forward, AACAGTCCCACTGGTCGTAGAT (SEQ ID NO: 12)
SREBF1 Reverse, TGTTGCAGAAAGCGAATGTAGT (SEQ ID NO: 13)
ACTB Forward, AGGCACTCTTCCAGCCTTCC (SEQ ID NO: 14)
ACTB Reverse, GCACTGTGTTGGCGTACAGG (SEQ ID NO: 15)
MMP-9 Forward, TTGACAGCGACAAGAAGTGG (SEQ ID NO: 16)
MMP-9 Reverse, GCCATTCACGTCGTCCTTAT (SEQ ID NO: 17)
MCP-1 Forward, CCCCAGTCACCTGCTGTTAT (SEQ ID NO: 18)
MCP-1 Reverse, TGGAATCCTGAACCCACTTC (SEQ ID NO: 19)
JNK1 Forward, TGCCTGACTCACACTCAAGG (SEQ ID NO: 20)
JNK1 Reverse, GGCTTCTCAGCAACTGAACC (SEQ ID NO: 21)
ABCA1 Forward, AACAGTTTGTGGCCCTTTTG (SEQ ID NO: 22)
ABCA1 Reverse, AGTTCCAGGCTGGGGTACTT (SEQ ID NO: 23)

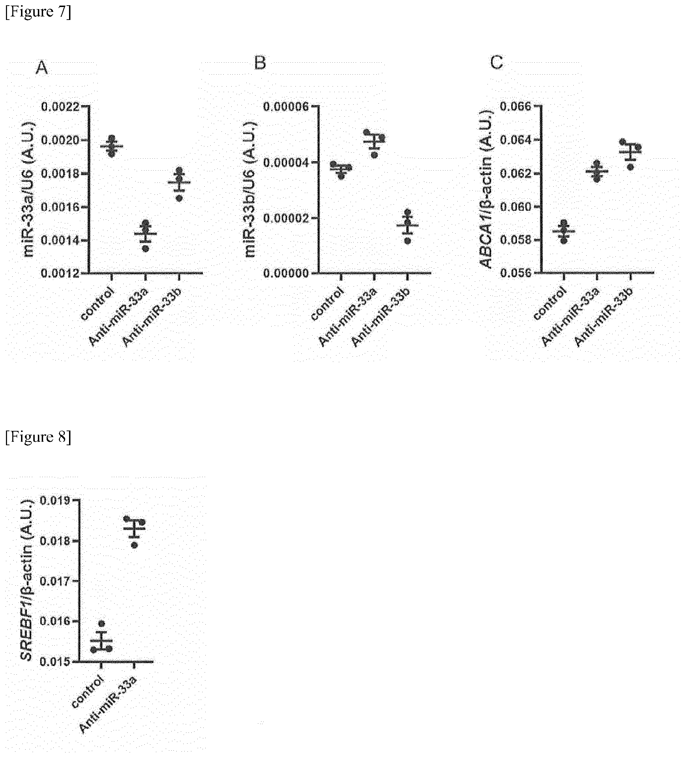

As shown in Fig. 7, strong suppression was observed in the expression level of miR-33a in the 33a-2-AmNA (12) introduction group and in the expression level of miR33b in the 33b-2-AmNA (12) introduction group. On the other hand, in the 33a-2-AmNA (12) introduction group, induction of the expression level of miR-33b was observed. A value of (reducing rate of miR-33a level)/(reducing rate of miR-33b level) was 0.52. Regarding a mRNA expression level of ABCA1, which is a target gene of miR-33a and miR-33b, all oligonucleotides showed a remarkable increase, confirming that they suppress miR-33a or b.

As shown in Fig. 8, in the 33a-2-AmNA (12) introduction group, induction of SREBF1 mRNA expression level was observed. Since miR-33b is present in an intron of a SREBP1 gene, an increased expression of the SREBF1 gene is thought to be a factor that induces the expression of miR-33b when the 33a-2-AmNA (12) introduction group, that is, a miR-33a antisense oligonucleotide, is processed.

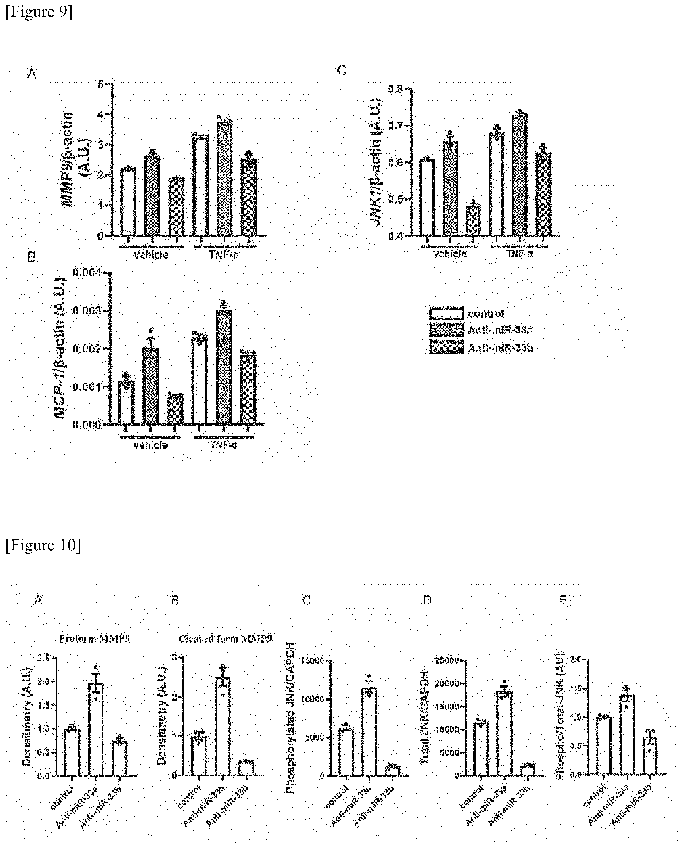

Regarding changes in MMP-9 mRNA, MCP-1 mRNA and JNK1 mRNA expression levels, the mRNA expression levels were measured using the above-described method when TNFα was not stimulated (vehicle) or when TNFαwas stimulated. As a result, as shown in Fig. 9, in the 33b-2-AmNA (12) administration group, all the mRNA expression levels were suppressed, whereas in the 33a-2-AmNA (12) administration group, all the mRNA expression levels increased.

Changes in MMP-9 protein and phosphorylated JNK protein levels were measured by Western blotting and gelatin zymography using the above-described method. As a result, as shown in Fig. 10, in the 33b-2-AmNA (12) administration group, the MMP-9 activity and the phosphorylated JNK protein expression level were suppressed, showing an inhibitory effect on macrophage activation, whereas in the 33a-2-AmNA (12) administration group, the MMP-9 activity and the phosphorylated JNK protein expression level were enhanced, showing an enhancing effect on macrophage activation.

As described above, regarding the inhibitory effect of 33b-2-AmNA (12) on the macrophage activation, which is a part of a pathological condition-forming mechanism in the mouse aneurysm model, it was clarified that the same effect was exhibited in human cells, showing that the miR-33b inhibiting substance is useful in treatment of human arterial aneurysms.

In the present specification, unless specifically stated otherwise, the use of a singular form includes a plural form. In the present specification, unless specifically stated otherwise, the use of "or" means "and/or." Further, the use of the term "including" and its other forms such as "includes" and "included" are not limiting. Further, unless specifically stated otherwise, the term "element" or the like includes an element that include one unit and an element that includes more than one subunit.

Section headings used herein are for organizational purposes only and should not be construed as limiting the subject matter described. All documents or portions of documents cited in this application, including, but not limited to, patents, patent applications, reports, books and articles, are expressly incorporated herein by reference, with respect to the portions of the document discussed herein, and in their entirety.

Unless a specific definition is given, the nomenclature used in connection with analytical chemistry, synthetic organic chemistry, and medical chemistry and pharmaceutical chemistry, and their procedures and techniques described herein are those that are well known in the art and are commonly used. Standard techniques can be used for chemical synthesis and chemical analysis as used herein. Where permitted, all patents, patent applications, published patent applications and other publications referred to throughout the disclosure of this specification, and GenBank accession numbers and relevant sequence information as well as other data available through databases such as the National Center for Biotechnology Information (NCBI), are incorporated by reference with respect to portions of the documents discussed herein, and in its entirety.

Further, this specification is filed together with a sequence listing in electronic format. However, the information of the sequence listing described in the electronic format is incorporated herein by reference in its entirety.

## Claims

1. A prophylactic or therapeutic agent for an aneurysm comprising a miR-33b inhibiting substance as an active ingredient.

2. The prophylactic or therapeutic agent according to claim 1, wherein the miR-33b inhibiting substance is a substance reducing miR-33b level.

3. The prophylactic or therapeutic agent according to claim 2, wherein the substance reducing miR-33b level is a nucleic acid.

4. The prophylactic or therapeutic agent according to claim 3, wherein the substance reducing miR-33b level is an antisense oligonucleotide, a siRNA or a shRNA against miR-33b.

5. The prophylactic or therapeutic agent according to any one of claims 2 to 4, wherein a reducing rate of miR-33b level of the substance reducing miR-33b level is higher than a reducing rate of miR-33a level of the substance reducing miR-33b level.

6. The prophylactic or therapeutic agent according to any one of claims 2 to 5, wherein the substance reducing miR-33b level is an antisense oligonucleotide against miR-33b.

7. The prophylactic or therapeutic agent according to claim 6, wherein the antisense oligonucleotide against miR-33b has a base sequence that (1) is formed of 7 to 20 nucleotide residues and (2) is 90% or more complementary to an equal length portion of miR-33b having a base sequence of SEQ ID NO: 1, and does not have a mismatch with 9th and 10th nucleobases counting from a 5' end of a base sequence of miR-33b described in SEQ ID NO: 1:
(SEQ ID NO: 1) GUGCAUUGCUGUUGCAUUGC

8. The prophylactic or therapeutic agent according to claim 7, wherein the antisense oligonucleotide against miR-33b comprises a nucleobase sequence described in AACAGCA ATGCA (SEQ ID NO: 5) wherein C may be 5-methylcytosine (M).

9. The prophylactic or therapeutic agent according to claim 8, wherein the antisense oligonucleotide is a modified oligonucleotide.

10. The prophylactic or therapeutic agent according to claim 9, wherein at least one nucleoside forming the modified oligonucleotide comprises a modified sugar.

11. The prophylactic or therapeutic agent according to claim 10, wherein the modified sugar is selected from a sugar moiety of AmNA: 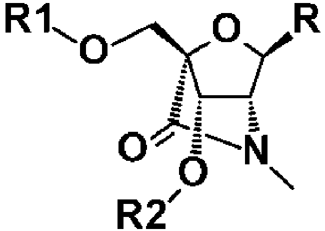 AmNA
wherein, R is a nucleobase, and R1 and R2 each independently indicate a phosphate group that may be substituted.

12. The prophylactic or therapeutic agent according to claim 11, wherein the modified oligonucleotide is a modified oligonucleotide represented by the following formula:
Aas Ads Mas Ads Gas Cds Aas Ads Tas Gds Mas Ad,
wherein nucleobases are represented according to the following symbols:
A = adenine, T = thymine, G = guanine, C = cytosine, M = 5-methylcytosine,
sugar moieties are represented according to the following symbols:
a = a sugar moiety of the above AmNA, d = 2'-deoxyribose,
and internucleoside linkages are represented according to the following symbol:
s = phosphorothioate.

13. The prophylactic or therapeutic agent according to claim 12, wherein the modified oligonucleotide is a modified oligonucleotide represented by the following formula:

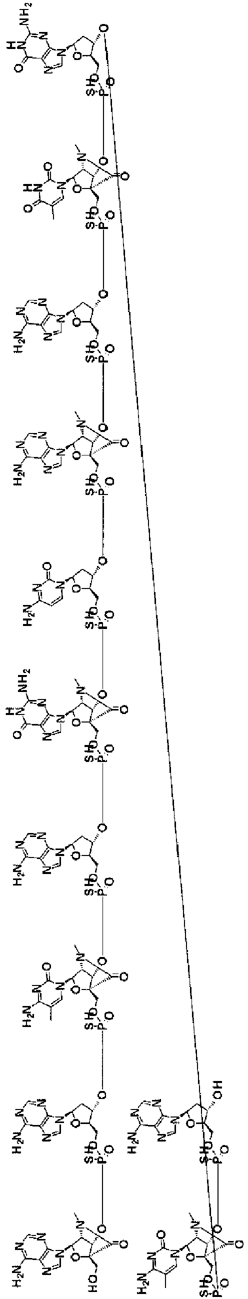

14. The prophylactic or therapeutic agent according to any one of claims 1 to 13, wherein the aneurysm is an aortic aneurysm or a cerebral aneurysm.

15. An antisense oligonucleotide against miR-33b comprising a nucleobase sequence described in AACAGCAATGCA (SEQ ID NO: 5).

16. The antisense oligonucleotide according to claim 15, wherein the antisense oligonucleotide is a modified oligonucleotide.

17. The antisense oligonucleotide according to claim 16, wherein at least one nucleoside forming the modified oligonucleotide comprises a modified sugar.

18. The antisense oligonucleotide according to claim 17, wherein the modified sugar is selected from a sugar moiety of AmNA: 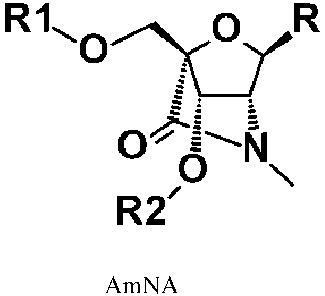 wherein, R is a nucleobase, and R1 and R2 each independently indicate a phosphate group that may be substituted.

19. The antisense oligonucleotide according to any one of claims 16 to 18, wherein at least one nucleoside forming the modified oligonucleotide comprises a modified nucleobase.

20. The antisense oligonucleotide according to claim 19, wherein the modified nucleobase is 5-methylcytosine.

21. The antisense oligonucleotide according to any one of claims 16 to 20, wherein a modified internucleoside linkage of the modified oligonucleotide is a phosphorothioate internucleoside linkage.

22. The antisense oligonucleotide according to any one of claims 16 to 21, wherein the modified oligonucleotide is a modified oligonucleotide represented by the following formula:
Aas Ads Mas Ads Gas Cds Aas Ads Tas Gds Mas Ad,
wherein nucleobases are represented according to the following symbols:
A = adenine, T = thymine, G = guanine, C = cytosine, M = 5-methylcytosine,
sugar moieties are represented according to the following symbols:
a = a sugar moiety of the above AmNA, d = 2'-deoxyribose,
and internucleoside linkages are represented according to the following symbol:
s = phosphorothioate.

23. The antisense oligonucleotide according to claim 22, wherein the modified oligonucleotide is represented by the following formula:

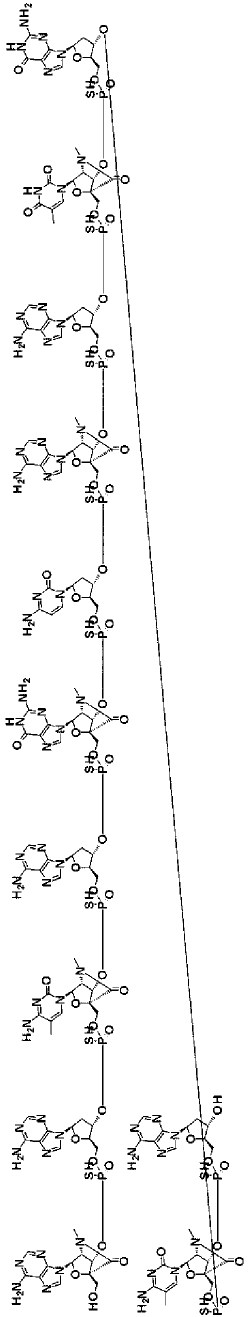

24. A pharmaceutical composition comprising: the antisense oligonucleotide according to any one of claims 15 to 23 or a pharmaceutically acceptable salt thereof; and a pharmaceutically acceptable carrier.

25. Use of a miR-33b inhibiting substance in manufacturing a prophylactic or therapeutic agent for an aneurysm.
